# DEMANDE DE BREVET EUROPEEN

(11) **EP 2 385 093 A1**
(43) Date de publication de la demande: **09.11.2011**
(21) Numéro de dépôt: 11290212.7
(22) Date de dépôt: 29.04.2011
(51) Int. Cl.: C10G 3/00, C10G 50/00, C07C 1/24, C07C 2/08, C07C 11/04

(54) **Procédé flexible de transformation de l'éthanol en distillats moyens mettant en oeuvre un système catalytique homogène et un système catalytique héterogène**

(30) Priorité: 06.05.2010 FR 1001954
(71) Demandeur: IFP Energies nouvelles, 92852 Rueil-Malmaison Cedex (FR)
(72) Inventeur: Guillon, Emmanuelle, 69390 Vourles (FR); Cadran, Nicolas, 69600 Oullins (FR); Touchais, Natacha, 38200 Vienne (FR); Bournay, Laurent, 69440 Chaussan (FR); Magna, Lionel, 69007 Lyon (FR); Olivier-Bourbigou, Hélène, 69230 St. Genis Laval (FR)

(57) **Abrégé**

L'invention décrit un procédé de production de bases hydrocarbonées distillats moyens à partir d'une charge éthanol produite à partir de source renouvelable issue de la biomasse comprenant une étape de purification de ladite charge, une étape de déshydratation de ladite charge purifiée en un effluent majoritairement éthylènique comprenant de l'eau, au moins une étape de séparation de l'eau, une première étape d'oligomérisation de l'effluent majoritairement éthylènique en au moins un effluent oléfinique comprenant au moins 80% poids d'oléfines ayant un nombre d'atomes de carbone supérieur ou égal à 4, en présence d'un catalyseur homogène comprenant au moins un composé de nickel bivalent, une deuxième étape d'oligomérisation produisant des bases hydrocarbonées distillats moyens en présence d'un catalyseur amorphe ou zéolithique ayant au moins des ouvertures de pores contenant 10 ou 12 atomes d'oxygènes, produisant des bases hydrocarbonées distillats moyens, et une étape de fractionnement.

## Description

### Domaine de l'invention

La présente invention concerne la transformation d'éthanol et plus particulièrement de bioéthanol en base carburants.

Elle concerne plus particulièrement un procédé catalytique flexible de transformation de l'éthanol en distillats moyens.

### Art Antérieur

La demande pour une utilisation de la biomasse en remplacement partiel des ressources pétrolières pour la synthèse de carburants va croissant. Ainsi l'utilisation de bioéthanol pour la synthèse de bases pour carburants reçoit un intérêt de plus en plus marqué.

Le bioéthanol est de l'éthanol d'origine agricole, c'est à dire qu'il est produit à partir de source renouvelable issue de la biomasse telle que par exemple les matières vivantes végétales.
La majeure partie de l'éthanol est produit par fermentation des sucres contenus dans les matières premières d'origine végétale. A partir des plantes sucrières, la première étape de la transformation consiste à obtenir un jus sucré par extraction à l'eau chaude pour la betterave ou par broyage et pressage pour la canne à sucre. Après concentration éventuelle, ces jus ou sirop, sont introduits dans des fermenteurs où se déroule la transformation biologique des sucres en éthanol avec la coproduction de CO₂ sous l'action de microorganismes (levure). Les vins obtenus contiennent environ 10% d'alcool dans l'eau. Une étape de distillation permet de parvenir à la composition azéotropique du binaire éthanol/eau (8% d'eau). Afin d'obtenir une déshydratation complète, un passage sur tamis moléculaire est nécessaire. Dans les plantes céréalières (maïs, blé), les sucres fermentescibles en éthanol sont présents sous la forme d'un polymère appelé amidon, pour les libérer une étape préalable d'hydrolyse catalysée par des enzymes est nécessaire.
De nouvelles technologies sont en cours de développement afin de permettre la transformation de la biomasse lignocellulosique (bois, herbe, paille et autres déchets agricoles, etc.) en bioéthanol.
L'éthanol est utilisable comme biocarburant dans les moteurs à essence. Ce produit présente des avantages notables : indice d'octane élevé, une miscibilité en toute proportion dans les essences et une densité voisine. Il s'agit d'un vecteur énergétique issu de l'agriculture et appartenant à la famille des énergies renouvelables. Il existe plusieurs types de carburants contenant de l'éthanol la plupart sont des mélanges d'essence et d'éthanol à différentes proportions. On les désigne par la lettre E suivie du pourcentage d'éthanol dans le mélange : par exemple du E85 représente un carburant contenant 85% d'éthanol et 15% d'essence. Dans cette nomenclature, E100 désigne l'éthanol pur. On trouve ainsi du E5, E7, E10, E15, E20, E85, E95, E100 en fonction du pays dans lequel on se trouve et de l'utilisation que l'on veut en faire.
En France, la commercialisation de l'E85 aux particuliers, légalement nommé Superéthanol, est officielle depuis le 1 er janvier 2007.
En Europe, les pétroliers continuent à transformer l'éthanol en ETBE (éthyl tertio butyl éther) qui peut être incorporé à l'essence jusqu'à hauteur de 15 %. L'ETBE présente l'avantage d'être mieux adapté aux moteurs. En effet, l'incorporation directe de l'éthanol à l'essence pose certaines difficultés techniques : le mélange essence/éthanol a une pression de vapeur plus élevée et tolère mal la présence de traces d'eau. Ces difficultés peuvent être surmontées par une reformulation des bases essence et par l'élimination des traces d'eau dans les cuves. Néanmoins, l'ETBE est moins vertueux pour l'environnement. Le parc automobile européen se caractérise par la forte proportion de motorisation diesel, il en résulte que le gazole est consommé en très forte proportion par rapport à l'essence. Les biocarburants incorporables au pool gazole sont donc particulièrement apprécié en Europe.

L'utilisation de l'éthanol est essentiellement destinée à la production d'essence, et non à la production de gazoles et kérosènes. Une autre voie très prometteuse est l'utilisation de l'éthanol comme biocarburant dans les moteurs diesel. Le biocarburant E-Diesel est un mélange qui se compose de diesel entre 85% et 95%, d'éthanol anhydre (sans eau) et un package d'additif spécialement réalisé pour la stabilité du mélange et pallier certains des inconvénients du bioethanol comme par exemple son faible indice de cétane, son faible pouvoir lubrifiant.
Mélanger le diesel conventionnel avec de l'éthanol et l'additif améliore le fonctionnement de la combustion et augmente légèrement la volatilité du combustible. Le résultat principal est la réduction des émissions de gaz polluants réglementés tels que les particules (PM10) et les fumées. Cette diminution se doit au contenu en oxygène du biocarburant qui limite la formation des particules lors de la combustion du carburant. En effet ces molécules oxygénées permettent une amélioration sensible de la qualité de la combustion par la présence de comburant à l'endroit même où la réaction d'oxydation se fait. Le mélange du bioethanol avec le diesel a aussi pour conséquence et inconvénient principal la réduction du point d'éclair.

La transformation de l'éthanol en hydrocarbures est donc une voie intéressante pour valoriser les ressources renouvelables vers des carburants.
La littérature est très riche sur la transformation d'alcools de type méthanol en oléfines ou aromatiques pour produire une coupe essence sur des catalyseurs acides, souvent zéolithiques.
La production d'éthylène à partir d'éthanol est un procédé connu, qui a été développé à l'échelle industrielle sur quelques unités. Ainsi des unités de déshydratation d'éthanol en éthylène ont été construites au Brésil durant les années 1970, suite à la crise pétrolière. L'éthanol est converti de façon catalytique en éthylène à partir de 300°C. Les catalyseurs utilisés peuvent être de nature différentes : alumine activée, silice alumine,...
Scientific Design a développé sa propre technologie de déshydratation de l'éthanol en éthylène et suite au développement d'un nouveau catalyseur, introduit sur une unité industrielle a publié un article ("Ethylene from Ethanol", N.K. Kochar, R. Merims, and A.S. Padia, CEP, Juin 1981). Les brevets US 4,232,179, US 4,396,789, US 4,234,752, US 4,396,789, US 4,698,452 peuvent être également cités.

Un objectif de la présente invention est de fournir un procédé de production de bases hydrocarbonées distillats moyens (gazole et/ou kérosène) et de préférence de bases kérosène incorporables au pool carburant et à des rendements élevés, à partir d'éthanol produit à partir de source renouvelable issue de la biomasse également appelé bioéthanol.

Le procédé selon l'invention mettant en oeuvre un enchaînement comprenant une étape de transformation d'une charge bioéthanol aqueuse produit à partir de source renouvelable issue de la biomasse en un effluent majoritairement éthylènique suivie de deux étapes d'oligomérisation, lesdites deux étapes utilisant des systèmes catalytiques spécifiques, permet de maximiser la production en bases distillats moyens et en particulier en kérosène, ce qui constitue à la fois un atout pour le raffineur, et un avantage du point de vue du développement durable.

### Objet de l'invention

La présente invention décrit donc un procédé de production de bases hydrocarbonées distillats moyens et de préférence en base hydrocarbonée kérosène à partir d'une charge éthanol produite à partir de source renouvelable issue de la biomasse, ledit procédé comprenant au moins :
a) une étape de purification de ladite charge éthanol,
b) une étape de déshydratation de ladite charge éthanol purifiée issue de l'étape a) en un effluent majoritairement éthylènique comprenant de l'eau, ladite étape opérant en présence d'un catalyseur acide amorphe ou d'un catalyseur acide zéolithique ,
c) au moins une étape de séparation de l'eau présente dans ledit effluent majoritairement éthylènique issu de l'étape b),
d) une première étape d'oligomérisation d'au moins une partie de l'effluent majoritairement éthylènique issu de l'étape c) en au moins un effluent oléfinique comprenant au moins 80% poids d'oléfines ayant un nombre d'atomes de carbone supérieur ou égal à 4, en présence d'un catalyseur homogène comprenant au moins un composé de nickel bivalent, les pourcentages poids étant exprimés en pourcentages poids par rapport à la masse totale des oléfines contenues dans ledit effluent oléfinique produit,
e) une deuxième étape d'oligomérisation d'au moins une partie de l'effluent issu de l'étape d) produisant des bases hydrocarbonées distillats moyens en présence d'un catalyseur amorphe ou zéolithique, ledit catalyseur zéolithique ayant au moins des ouvertures de pores contenant 10 ou 12 atomes d'oxygènes,
f) une étape de fractionnement de l'effluent issu de l'étape e) d'oligomérisation.

### Description détaillée de l'invention

### Charge

La charge traitée dans le procédé selon l'invention est une charge éthanol produite à partir de source renouvelable issue de la biomasse et sera en conséquence appelée "charge bioéthanol" dans la suite de la description.
Ladite charge bioéthanol est une charge produite par voie biologique, plus précisément par fermentation de sucres issus par exemple des cultures de plantes sucrières comme la canne à sucre (saccharose, glucose, fructose, et sucrose), des betteraves, ou encore des plantes amylacées (amidon) ou de la biomasse lignocellulosique ou de cellulose hydrolysée (glucose majoritaire et xylose, galactose), contenant des quantités variables d'eau.
Ladite charge est avantageusement obtenue par fermentation à partir de trois sources : 1) le sucrose de canne ou de betterave, 2) l'amidon présent dans les céréales et les tubercules et 3) la cellulose et l'hémicellulose présentes dans le bois, les herbes et autres biomasses lignocellulosiques, l'amidon, la cellulose et l'hémicellulose devant être hydrolysées en sucres avant de subir une étape de fermentation.

La charge bioéthanol utilisée selon l'invention contient donc majoritairement de l'éthanol, à hauteur de plus de 50% poids et de préférence plus de 70% poids et contient également avantageusement une teneur en eau supérieure à 2% poids, de préférence supérieure à 5% poids et de manière préférée supérieure à 10% poids, une teneur en impureté cationique telle que par exemple les ions Na+, Ca2+, Mn2+, Fe2+, Cu2+, Zn2+ avantageusement inférieure à 0,5% poids, une teneur en impureté anionique telle que par exemple les ions Cl-, sulfate, nitrite, nitrate, phosphates avantageusement inférieure à 0,5% poids, une teneur en autres alcools tels que par exemple le méthanol ou le butanol avantageusement inférieure à 10% poids, et de préférence inférieure à 5% poids, une teneur en composés oxygénés autres que les alcools tels que par exemple les éthers, les acides, les cétones, les aldéhydes et/ou les esters avantageusement inférieure à 1% poids et une teneur en azote et en soufre avantageusement inférieure à 0,5% poids, les pourcentages poids étant exprimés par rapport à la masse totale de ladite charge.

Conformément à l'étape a) du procédé selon l'invention, la charge subit une étape de purification de manière à éliminer ies impuretés cationique et anionique ainsi qu'au moins une partie des composés oxygénés pour limiter la désactivation du catalyseur de déshydratation placé en aval.

L'étape de purification est avantageusement mise en oeuvre par des moyens connus de l'homme du métier, tel que par exemple l'utilisation d'au moins une résine, l'adsorption des impuretés et des composés oxygénés sur solides choisis parmi les tamis moléculaires, le charbon actif, l'alumine et les zéolithes et la distillation pour produire une coupe purifiée de bioéthanol et une coupe comprenant les impuretés organiques afin d'obtenir une charge purifiée répondant au niveau d'impuretés compatibles avec le catalyseur de déshydratation.

Une étape de prétraitement peut avantageusement être mise en oeuvre par hydrogénation des composés insaturés oxygénés en présence d'un catalyseur à base de nickel, ladite étape de pré-traitement étant mise en oeuvre avant ou après l'étape de purification et de préférence après.

Conformément à l'étape b) du procédé selon l'invention, la charge éthanol purifiée issue de l'étape a) subit une étape de déshydratation en un effluent majoritairement éthylènique comprenant de l'eau, ladite étape opérant en présence d'un catalyseur de déshydratation connu de l'homme du métier, en particulier d'un catalyseur acide amorphe ou d'un catalyseur acide zéolithique.

Dans le cas où le catalyseur utilisé dans l'étape b) de déshydratation est un catalyseur zéolithique, ledit catalyseur comprenant au moins une zéolithe choisie parmi les zéolithes ayant au moins des ouvertures de pores contenant 10 ou 12 atomes d'oxygènes (10MR ou 12 MR). Il est connu en effet de définir la taille des pores des zéolithes par le nombre d'atomes d'oxygène formant la section annulaire des canaux des zéolithes, appelés "member ring" ou MR en anglais. De manière préférée, ledit catalyseur zéolithique comprend au moins une zéolithe présentant un type structural choisi parmi les types structuraux MFI, FAU, MOR, FER, et BEA.
La zéolithe mise en oeuvre dans le catalyseur utilisé dans l'étape b) du procédé selon l'invention peut avantageusement être modifiée par désalumination ou désilication selon toute méthode de désalumination ou désilication connue de l'homme du métier.

Dans le cas où le catalyseur utilisé dans l'étape b) de déshydratation est un catalyseur acide amorphe, ledit catalyseur comprend au moins un oxyde réfractaire poreux choisi parmi l'alumine, l'alumine activée par un dépôt d'acide minéral et la silice alumine.

Ledit catalyseur de déshydratation amorphe ou zéolithique utilisé dans l'étape b) du procédé selon l'invention peut avantageusement également comprendre au moins une matrice de type oxyde également appelé liant. On entend par matrice selon l'invention, une matrice amorphe ou mal cristallisée.
Ladite matrice est avantageusement choisie parmi les éléments du groupe formé par les argiles (telles que par exemple parmi les argiles naturelles telles que le kaolin ou la bentonite), la magnésie, les alumines, les silices, les silice-alumines, les aluminates, l'oxyde de titane, l'oxyde de bore, la zircone, les phosphates d'aluminium, les phosphates de titane, les phosphates de zirconium, et le charbon. De préférence ladite matrice est choisie parmi les éléments du groupe formé par les alumines, les silices et les argiles.
Dans un mode préféré, le liant possède une texture macroporeuse comme décrit dans la demande de brevet US2009/088595.

Le catalyseur de déshydratation utilisé dans l'étape b) du procédé selon l'invention est avantageusement mis en forme sous la forme de grains de différentes formes et dimensions. Il est avantageusement utilisé sous la forme d'extrudés cylindriques ou polylobés tels que bilobés, trilobés, polylobés de forme droite ou torsadée, mais peut éventuellement être fabriqué et employé sous la forme de poudre concassée, de tablettes, d'anneaux, de billes, de roues, de sphères. De préférence, ledit catalyseur est sous forme d'extrudés ou de billes.

L'étape b) de déshydratation du procédé selon l'invention opère avantageusement à une température comprise entre 250 et 600°C, de préférence entre 300 et 600°C et de manière préférée entre 300 et 500°C, à une pression absolue comprise entre 0,1 et 5MPa, de préférence entre 0,1 et 2,5 MPa et de manière préférée entre 0,1 et 1MPa et à une vitesse pondérale horaire comprise entre 0,1 et 50 h⁻¹ et de préférence entre 0,5 et 15h⁻¹. La vitesse pondérale horaire est définie comme étant le rapport du débit massique de la charge sur la masse de catalyseur.

Dans l'étape b) du procédé selon l'invention, les catalyseurs utilisés et les conditions opératoires sont choisis de manière à maximiser la production d'éthylène. La réaction de déshydration mise en oeuvre dans l'étape b) du procédé selon l'invention est la suivante: 2 C₂H₅OH → ₂CH₂=CH₂ + 2 H₂O

Ladite étape b) produit un effluent majoritairement éthylènique. On entend par effluent majoritairement éthylènique produit lors de l'étape b), un effluent comprenant au moins 95%, de préférence au moins 97% et de manière préférée, au moins 98% poids d'éthylène par rapport à la masse totale des composés carbonés formés et présents dans ledit effluent produit à ladite étape b). Outre la présence majoritaire d'éthylène, ledit effluent carboné peut également comprendre d'autres composés hydrocarbonés, hydroxycarbonés ou carbonés en une proportion très minoritaire. En particulier, ledit effluent carboné comprend avantageusement moins de 5%, de préférence moins de 3% et de manière préférée moins de 2% poids de composés ayant un nombre d'atomes de carbone supérieur ou égal à 3 et de composés oxygénés tels que par exemples le CO2, le CO, de diéthyléther ou l'acétaldéhyde, les pourcentages étant exprimés en pourcentages poids par rapport à la masse totale des composés carbonés formés et présents dans ledit effluent produit à ladite étape b).

La conversion de la charge bioéthanol dans l'étape b) est avantageusement supérieure à 90%, de préférence 95% et de manière préférée supérieure à 98%.

On entend par conversion de la charge bioéthanol, le rapport de la différence entre le débit massique de la charge éthanol (C₂H₅OH) en entrée et le débit massique de la charge éthanol (C2H5OH) en sortie de l'étape b) sur le débit massique de la charge éthanol en entrée.
La transformation de la charge s'accompagne de la désactivation du catalyseur par cokage et/ou par adsorption de composés inhibiteurs. Le catalyseur devra donc subir périodiquement une étape de régénération décrite ci dessous.

Un diluant à base d'eau ou de paraffines provenant d'une charge extérieure au procédé selon l'invention tel que par exemple des paraffines ayant un nombre de carbone compris entre 2 et 8 peut avantageusement être ajouté à la charge bioéthanol après purification dans un rapport molaire diluant sur charge, avantageusement compris entre 0,5 à 20 dans le but de stabiliser le catalyseur de l'étape b) du procédé selon l'invention.

L'étape b) de déshydratation du procédé selon l'invention de ladite charge purifiée, en un effluent majoritairement éthylènique, est avantageusement mise en oeuvre dans au moins un réacteur en lit fixe par exemple selon l'enseignement du brevet US4396789, en lit mobile ou en lit fluidisé.

Dans le cas ou l'étape b) est mise en oeuvre en lit fluidisé, le catalyseur prendra la forme de billes, de taille avantageusement inférieure à 500 microns et de préférence inférieure à 300 microns.

Dans le cas où l'étape b) est mise en oeuvre en lit fixe, la régénération du catalyseur utilisé dans ladite étape b) est avantageusement réalisée par oxydation du coke et des composés inhibiteurs sous flux d'air par exemple en utilisant une recirculation de l'air de combustion avec ou sans eau afin de diluer l'oxygène et maîtriser l'exothermie de régénération. Dans ce cas, on peut avantageusement ajuster la teneur en oxygène en entrée du réacteur par un appoint d'air. La régénération a lieu à pression entre la pression atmosphérique (0 bar relatif) et la pression de réaction. La température de régénération est avantageusement choisie entre 400 et 600°C ; elle peut avantageusement varier en cours de régénération. La fin de la régénération est détectée quand il n'y a plus de consommation d'oxygène, signe d'une combustion totale du coke.

Dans le cas où l'étape b) est mise en oeuvre en lit mobile ou fluidisé comme décrit dans le brevet US 4 134 926, la régénération du catalyseur s'effectue en continu.

Conformément à l'étape c) du procédé selon l'invention, l'effluent majoritairement éthylénique issu de l'étape b) subit au moins une étape de séparation de l'eau présente dans l'effluent produit lors de l'étape b).
De préférence, ledit effluent issu de l'étape b) subit au moins une étape de séparation de l'eau présente dans ledit effluent, ladite étape de séparation étant suivie d'au moins une étape de purification dudit effluent préalablement séparé de l'eau. Ladite étape c) du procédé selon l'invention permet d'éliminer les impuretés néfastes pour les catalyseurs d'oligomérisation utilisés pour les mises en oeuvre des étapes d) et e) placés en aval et en particulier ladite étape c) permet l'élimination des composés oxygénés présents dans ledit effluent.

L'étape c) du procédé selon l'invention mettant en oeuvre au moins une étape de séparation de l'eau et/ éventuellement au moins une étape de purification dudit effluent majoritairement éthylénique issu de ladite étape b) peut avantageusement être mise en oeuvre par toute méthode connue de l'homme du métier telle que par exemple la combinaison successive d'un traitement dans une colonne de lavage à l'eau, puis passage dans une colonne d'absorption à la MDEA (méthyldiéthylamine) ou autre amine et dans une colonne de lavage à la soude ou tout autre moyen connu de l'homme de l'art. Des sécheurs peuvent avantageusement être mis en oeuvre de manière à atteindre une teneur en eau compatible avec les catalyseurs d'oligomérisation utilisés en aval dans les étapes d) et e) d'oligomérisation. La teneur en eau de l'effluent envoyé dans de l'étape d) d'oligomérisation est avantageusement comprise entre 0 et 500 ppm et de préférence inférieure à 100 ppm.

Au moins une partie de l'effluent aqueux éliminée lors de l'étape c) est avantageusement recyclée en amont de l'étape b) de déshydratation de ladite charge purifiée, servant de diluant de la charge bioéthanol purifiée.

Conformément à l'étape d) du procédé selon l'invention, l'effluent majoritairement éthylènique issu de l'étape c) de séparation est envoyée dans une première étape d'oligomérisation d) dans laquelle ledit effluent majoritairement ethylènique est oligomérisé, en présence d'un catalyseur homogène comprenant au moins un composé de nickel bivalent, en au moins un effluent oléfinique comprenant au moins 80% poids et de préférence au moins 90% poids d'oléfines ayant un nombre d'atomes de carbone supérieur ou égal à 4, les pourcentages poids étant exprimés par rapport à la masse totale des oléfines contenues dans ledit effluent oléfinique produit.
Au moins une partie, c'est à dire au moins 50 % poids, de préférence au moins 90% poids dudit effluent issu de ladite étape c) et de manière préférée la totalité dudit effluent issu de ladite étape c) est soumis à ladite première étape d'oligomérisation.
Outre la présence majoritaire d'oléfines ayant un nombre d'atomes de carbone supérieur ou égal à 4, l'effluent oléfinique produit lors de la première étape d'oligomérisation d) comprend également avantageusement moins de 20%, de préférence moins de 10% poids d'éthylène (C2) n'ayant pas réagi lors de la première étape d'oligomérisation d), les pourcentages étant exprimés en pourcentages poids par rapport à la masse totale des oléfines contenues dans l'effluent produit.
L'objectif de ladite première étape d) d'oligomérisation est d'obtenir un effluent hydrocarboné oléfinique ayant un nombre d'atomes de carbone supérieur ou égal à 4 et en particulier, riche en oléfine ayant un nombre d'atomes de carbone compris entre 4 et 8.

La première étape d) d'oligomérisation conduit à la production d'un effluent oléfinique comprenant au moins 80% poids par rapport à la masse totale des oléfines contenues dans ledit effluent oléfinique, d'oléfines ayant un nombre d'atomes de carbone supérieur ou égal à 4. En particulier, ledit effluent est riche en hydrocarbures oléfiniques ayant un nombre d'atomes de carbone compris entre 4 et 8 et comprend également des hydrocarbures oléfiniques ayant au moins 9 atomes de carbone (C9+). Plus particulièrement, ledit effluent oléfinique produit lors de la première étape d'oligomérisation d), comprend avantageusement au moins 80% poids, de préférence au moins 90% poids, de composés oléfiniques ayant majoritairement un nombre d'atomes de carbones compris entre 4 et 8 et moins de 20% poids et de préférence moins de 10% poids, de composés oléfiniques ayant majoritairement un nombre d'atome de carbone supérieur ou égal à 9, les pourcentages poids étant exprimés par rapport à la masse totale des oléfines contenues dans ledit effluent oléfinique produit.

De préférence, la charge entrant dans le(s) réacteurs(s) mettant en oeuvre ladite première étape d'oligomérisation d) y est introduite en mélange avec une partie, de préférence la totalité d'un effluent léger hydrocarboné oléfinique ayant un nombre d'atomes de carbone majoritairement compris entre 2 et 4 (effluent léger oléfinique C2-C4), lequel est issu d'une étape de séparation optionnelle décrite ci dessous et préférentiellement mise en oeuvre entre lesdites étapes d'oligomérisation d) et e) selon l'invention.

Conformément à l'étape d) du procédé selon l'invention, le catalyseur utilisé dans la première étape d'oligomérisation d) est un catalyseur homogène comprenant au moins un composé de nickel bivalent, c'est-à-dire que le catalyseur est soluble dans la phase liquide composée de l'éthylène dissous et ses produits d'oligomérisation.
S'agissant d'une mise en oeuvre par catalyse homogène, l'Homme du métier pourra utilement se référer à l'enseignement des brevets US 7.235.703 et US 4.362.650.
Le catalyseur homogène utilisé dans l'étape d) d'oligomérisation du procédé selon l'invention comprend au moins un composé de nickel bivalent, éventuellement au moins un halogénure d'hydrocarbyl-aluminium et éventuellement au moins un acide organique de Brönsted. De préférence, le catalyseur peut également contenir au moins un anhydride d'acide carboxylique.

Les composés de nickel bivalent sont de préférence des composés solubles à plus d'un gramme par litre en milieu hydrocarboné, et plus particulièrement dans les réactifs et le milieu de réaction et de manière préférée, des carboxylates de nickel de formule générale (RCOO)2Ni où R est un radical hydrocarbyle, par exemple alkyle, cycloalkyle, alkényle, aryle, aralkyle ou alkaryle contenant jusqu'à 20 atomes de carbone, de préférence un reste hydrocarbyle de 5 à 20 atomes de carbone. Le radical R peut être substitué par un ou plusieurs atomes d'halogène, par un ou plusieurs groupes hydroxy, cétone, nitro, cyano ou autres groupes qui ne gênent pas la réaction. Les deux radicaux R peuvent aussi constituer un reste alkylène de 6 à 18 atomes de carbone. Les composés de nickel bivalent sont avantageusement choisis parmi les sels de nickel bivalent suivants : octoate, éthyl-2-hexanoate, décanoate, stéarate, oléate, salicylate et hydroxydécanoate, pris seuls ou en mélange et de préférence, le composé de nickel bivalent est l'ethyle-2-hexanoate de nickel.
Les halogénures d'hydrocarbyl-aluminium sont de préférence des dihalogénures d'hydrocarbyl-aluminium répondant à la formule AIRX₂, dans laquelle R est un radical hydrocarbyle et X un halogène choisi parmi le fluor, le chlore, le brome et l'iode, pris seuls ou en mélange. Les halogénures d'hydrocarbyl-aluminium sont avantageusement choisis parmi le dichloroéthylaluminium, le dichloroisobutylaluminium et le dibromoéthylaluminium. Ces dihalogénures d'hydrocarbylaluminium peuvent être avantageusement enrichis avec des trihalogénures d'aluminium (AIX₃) comme par exemple le trichlorure d'aluminium.

Les composés acides organiques de Brönsted répondent de préférence à la formule HY, où Y est un anion organique, par exemple carboxylique, sulfonique ou phénolique. Lesdits composés ont de préférence un pKa à 20°C au maximum égal à 3 et sont choisis de préférence dans le groupe formé par les acides halogénocarboxyliques de formule RCOOH dans laquelle R est un radical alkyle halogéné et de préférence un radical alkyle halogéné renfermant au moins un atome d'halogène en alpha du groupe -COOH avec au total de 2 à 10 atomes de carbone.
On utilise de préférence un acide halogénoacétique de formule CXₚH₃₋ₚ-COOH dans laquelle X est le fluor, le chore, le brome ou l'iode, avec p entier de 1 à 3. A titre d'exemple, on peut citer les acides trifluoroacétique, difluoroacétique, fluoroacétique, trichloroacétique, dichoroacétique, chloroacétique. Ces exemples ne sont pas limitatifs, et on peut aussi utiliser les acides arylsulfoniques, alkylsulfoniques, fluoroalkylsulfoniques, l'acide picrique, l'acide nitroacétique.

Le catalyseur utilisé dans la première étape d) d'oligomérisation peut également contenir au moins un anhydride d'acide carboxylique de formule (RCO)₂O dans laquelle R est un radical hydrocarbyle pouvant avantageusement contenir un ou plusieurs atomes d'halogène. Les anhydrides d'acide carboxylique sont avantageusement choisis parmi les anhydrides octoïque, éthyl-2-hexanoïque, décanoïque, stéarique, oléique, trifluoroacétique, monofluoroacétique, trichloroacétique, monochloroacétique, pentafluoropropionique ou heptafluorobutyrique, pris seuls ou en mélange. De préférence, l'anhydride d'acide carboxylique est l'anhydride de l'acide trifluoroacétique.

Les différents composés constituant le catalyseur peuvent être mélangés dans un ordre quelconque. Cependant, il est préférable de mélanger d'abord le composé de nickel avec l'acide organique de Brönsted, puis d'introduire ensuite le composé d'aluminium.
Un préconditionnement du catalyseur peut être effectué avant la mise en contact du catalyseur avec l'éthylène. Le préconditionnement de la composition catalytique consiste à effectuer le mélange des trois composants dans un solvant hydrocarboné, par exemple un alcane ou un hydrocarbure aromatique, ou encore un hydrocarbure halogéné, ou encore de façon préférée les oléfines produites dans la réaction d'oligomérisation, sous agitation et sous atmosphère inerte, par exemple sous azote ou sous argon, à une température contrôlée comprise entre 0 et 80°C, de préférence entre 10 et 60°C, pendant une durée de 1 minute à 5 heures, de préférence de 5 minutes à 1 heure. La solution ainsi obtenue est ensuite transférée sous atmosphère inerte dans le réacteur d'oligomérisation.
Ce préconditionnement du catalyseur permet d'augmenter l'activité du catalyseur dans l'oligomérisation de l'éthylène.

Le catalyseur présent dans ladite unité opérant l'étape d'oligomérisation d) se présente sous forme liquide. En fonction de la composition chimique dudit catalyseur, les proportions pondérales de chacun des composants du catalyseur sont à contrôler lors de la synthèse du catalyseur. Le rapport molaire de l'halogénure d'hydrocarbylaluminium au composé de nickel, exprimé par le rapport Al/Ni, est de 2/1 à 50/1, et de préférence de 2/1 à 20/1.
Le rapport molaire de l'acide de Bronsted au composé de nickel est de 0,25/1 à 10/1, et de préférence de 0,25/1 à 5/1. Si le catalyseur comprend de l'anhydride d'acide carboxylique, le rapport molaire de l'anhydride d'acide carboxylique au composé de nickel est avantageusement compris entre 0,001/1 et 1/1, très avantageusement entre 0,01/1 et 0,5/1.

La première étape d) d'oligomérisation mise en oeuvre par catalyse homogène est avantageusement réalisée en continu : la solution catalytique est injectée dans l'unité opérant l'étape d'oligomérisation et l'éthylène y est injecté en continu. L'unité opérant ladite étape d'oligomérisation de l'éthylène par catalyse homogène comprend un ou plusieurs réacteurs de type parfaitement agité, en série, avec recycle d'une partie, au moins, de l'effluent du réacteur dans le réacteur, ce recyle ayant été avantageusement refroidi. Le recycle a un rôle de diluant thermique et d'extracteur de chaleur car la réaction est exothermique . Le recycle est interne à l'unité d'oligomérisation.
La première étape d'oligomérisation d) peut être mise en oeuvre dans un réacteur à un ou plusieurs étages de réaction en série, la charge majoritairement éthylènique et/ou la composition catalytique au préalable préconditionnée étant introduits en continu, soit dans le premier étage, soit dans le premier et un autre quelconque des étages.
Les conditions opératoires dans le(s) réacteur(s) opérant l'étape d'oligomérisation par catalyse homogène sont telles que la température est comprise entre -20 °C et +80 °C et la pression suffisante pour permettre l'existence d'une phase liquide dans le(s) réacteur(s). De manière préférée, la pression totale absolue dans le(s) réacteur(s) se situe entre 2 et 8 MPa.

Ledit effluent oléfinique issu de ladite première étape d'oligomérisation d) comprend de préférence au moins 80% poids et de préférence au moins 90% poids de composés oléfiniques ayant un nombre d'atomes de carbone supérieur ou égal à 4 et moins de 20% poids et de préférence moins de 10% poids d'éthylène (C2) n'ayant pas réagi lors de la première étape d'oligomérisation d), les pourcentages étant exprimés en pourcentages poids par rapport à la masse totale des oléfines contenues dans ledit effluent oléfinique produit.

A la sortie de la première étape d) d'oligomérisation, le système catalytique homogène se trouve en mélange avec l'effluent oléfinique produit lors de l'étape d) et l'éthylène qui n'a pas réagi. Dans un premier mode de réalisation, au moins une partie et de préférence la totalité dudit effluent oléfinique produit lors de la première étape d) d'oligomérisation est directement envoyée dans la deuxième étape e) d'oligomérisation.

Dans un second mode de réalisation, ledit effluent oléfinique produit lors de la première étape d) d'oligomérisation subit au moins une étape de traitement du système catalytique homogène et/ou au moins une étape de séparation optionnelle dudit effluent avant d'être envoyé dans la deuxième étape e) d'oligomérisation.

Dans ledit second mode de réalisation, on entend par étape de traitement du système catalytique homogène, une étape dans laquelle ledit système catalytique est désactivé et séparé du milieu réactionnel homogène et en particulier de l'effluent oléfinique issu de la première étape d) d'oligomérisation.
Ladite étape de traitement optionnelle du système catalytique homogène est avantageusement mise en oeuvre selon 3 méthodes :
1) soit par l'utilisation de masse de captation,
2) soit par traitement par une base et/ou un acide, de l'effluent oléfinique issu de la première étape d) d'oligomérisation, neutralisé ou non par une base,
3) soit par la séparation dudit l'effluent oléfinique issu de la première étape d) d'oligomérisation, neutralisé ou non par une base, en un premier effluent comprenant au moins une partie des composés oléfiniques C9+ et également le système catalytique homogène et en un deuxième effluent oléfinique exempt du système catalytique, ladite séparation étant suivie du traitement de l'effluent comprenant au moins une partie des composés C9+ et le système catalytique homogène par lavage acide et/ou basique ou par l'utilisation de masse de captation.

Dans le cas ou ladite étape de traitement du système catalytique homogène est avantageusement mise en oeuvre par l'utilisation de masse de captation, lesdites masses de captation sont avantageusement choisies parmi les alumines, les silices et les terres activées prises seules ou en mélange, le système catalytique homogène s'adsorbant sur la surface du solide constituant la masse de captation.

Dans le cas ou ladite étape de traitement du système catalytique homogène est avantageusement mise en oeuvre par traitement dudit effluent oléfinique, contenant ledit système catalytique homogène, par une base et/ou un acide, ledit traitement consiste avantageusement en l'ajout d'une solution aqueuse qui va permettre le transfert du système catalytique de la phase hydrocarbonée à la phase aqueuse. Par séparation de la phase aqueuse et de la phase hydrocarbonée, non miscibles, le système catalytique est éliminé de la phase hydrocarbure. Le traitement par une base et/ou un acide se fait avantageusement successivement à l'aide d'ammoniac et/ou d'une solution aqueuse de soude et/ou d'une solution aqueuse d'acide sulfurique.
Le traitement par lavage acide et/ou basique est avantageusement suivi d'une étape de séchage dudit effluent, ladite étape de séchage étant de préférence mise en oeuvre par l'utilisation de tamis moléculaire ou d'adsorbants visant l'élimination résiduelle d'eau.

Dans le cas ou ladite étape de traitement du système catalytique homogène est avantageusement mise en oeuvre selon la troisième méthode, la séparation dudit effluent oléfinique en un premier effluent comprenant au moins une partie des composés C9+, et également le système catalytique homogène et en un deuxième effluent oléfinique exempt du système catalytique, est avantageusement mise en oeuvre par toute méthode connue de l'homme du métier telle que par exemple la combinaison d'un ou plusieurs ballons séparateurs haute et/ou basse pression et haute et/ou basse température, et/ou d'étapes de distillation comprenant une ou plusieurs colonnes de distillation, et de préférence par la combinaison d'un ou plusieurs ballons séparateurs haute et/ou basse pression et haute et/ou basse température.
Selon la troisième méthode, ladite séparation est ensuite avantageusement suivie du traitement dudit l'effluent comprenant au moins une partie des composés C9+ et le système catalytique homogène par lavage acide et/ou basique ou par l'utilisation de masse de captation tels que définis ci dessus.
Dans le cas où le traitement est mis en oeuvre par lavage acide et/ou basique, il est avantageusement suivi d'une étape de séchage de l'effluent comprenant au moins une partie des composés C9+, ladite étape de séchage étant de préférence mise en oeuvre par l'utilisation de l'utilisation de tamis moléculaire ou d'adsorbants visant l'élimination résiduelle d'eau.

Dans ledit second mode de réalisation, au moins une étape de séparation optionnelle de l'effluent oléfinique produit lors de la première étape d) d'oligomérisation, ledit effluent ayant éventuellement subi une étape de traitement du système catalytique homogène, est avantageusement mise en oeuvre entre la première étape d'oligomérisation d) et la deuxième étape d'oligomérisation e). De préférence au moins une étape de séparation optionnelle est mise en oeuvre entre ladite étape de traitement du système catalytique homogène, lorsque que celle ci est mise en oeuvre, et la deuxième étape d'oligomérisation e). Cette étape permet la séparation dudit effluent oléfinique issu de la première étape d'oligomérisation d) ou issu de l'étape traitement du système catalytique homogène, en au moins un effluent oléfinique ayant un nombre d'atomes de carbone supérieur ou égal à 4 et en au moins un effluent léger oléfinique (C2-C4).
En particulier, ledit effluent oléfinique ayant un nombre d'atomes de carbone supérieur ou égal à 4 est riche en composés oléfiniques ayant un nombre d'atomes de carbone compris entre 4 et 8 et comprend également des composés oléfiniques ayant au moins 9 atomes de carbone (C9+).
Ladite étape de séparation peut avantageusement être mise en oeuvre par toute méthode connue de l'homme du métier telle que par exemple la combinaison d'un ou plusieurs ballons séparateurs haute et/ou basse pression et haute et/ou basse température, et/ou d'étapes de distillation comprenant une ou plusieurs colonne de distillation.
On entend par effluent léger oléfinique C2-C4, un effluent comprenant avantageusement au moins 50% poids et de préférence au moins 65% poids de composés oléfiniques ayant un nombre d'atomes de carbone compris entre 2 et 4 et comprenant également avantageusement moins de 50% poids et de préférence moins de 45% poids de composés oléfiniques ayant un nombre d'atomes de carbone supérieur ou égal à 5, les pourcentages poids étant exprimés par rapport à la masse totale d'oléfines dudit effluent léger oléfinique C2-C4 issu de la séparation optionnelle.
De préférence, la totalité dudit effluent léger oléfinique C2-C4 est recyclée dans la première étape d'oligomérisation d).
On entend par effluent oléfinique ayant un nombre d'atomes de carbone supérieur ou égal à 4, un effluent comprenant avantageusement au moins 80% poids, de préférence au moins 90% poids, de composés oléfiniques ayant un nombre d'atomes de carbones compris entre 4 et 8 (C4-C8) et moins de 20% poids et de préférence moins de 10% poids, de composés oléfiniques ayant un nombre d'atomes de carbones supérieure à 9 (C9+), les pourcentages poids étant exprimés par rapport à la masse totale des oléfines présentes dans ledit effluent oléfinique ayant un nombre d'atomes de carbone supérieur ou égal à 4.

Dans ledit second mode de réalisation et dans le cas où l'étape de traitement du système catalytique homogène est mise en oeuvre par l'utilisation de masse de captation ou par lavage acide et/ou basique, l'effluent oléfinique issu de ladite étape de traitement du système catalytique homogène peut avantageusement soit, être directement envoyé dans la deuxième étape d'oligomérisation e), sans subir une étape de séparation optionnelle intermédiaire, soit subir une étape de séparation optionnelle dans laquelle ledit effluent est avantageusement séparé en au moins un effluent oléfinique ayant un nombre d'atomes de carbone supérieur ou égal à 4 et en au moins un effluent léger oléfinique (C2-C4).
Dans le cas ou l'effluent oléfinique issu de ladite étape de traitement du système catalytique homogène subit ladite étape de séparation optionnelle, la totalité dudit effluent léger oléfinique C2-C4 issu de ladite étape de séparation est de préférence recyclée dans la première étape d'oligomérisation d).
Ledit effluent oléfinique ayant un nombre d'atomes de carbone supérieur ou égal à 4 et ledit effluent léger oléfinique (C2-C4) ont la signification définie ci dessus.
Selon une première variante, dans le cas ou l'effluent oléfinique issu de ladite étape de traitement du système catalytique homogène subit ladite étape de séparation optionnelle, la totalité de l'effluent oléfinique C4+ issu de ladite étape de séparation, comprenant les composés oléfiniques C4-C8 et les composés oléfiniques C9+, est avantageusement envoyée dans la deuxième étape d'oligomérisation e).
Selon une deuxième variante, l'effluent oléfinique C4+ est avantageusement séparé, dans une deuxième étape de séparation optionnelle, en au moins un effluent oléfinique ayant un nombre d'atomes de carbones compris entre 4 et 8 (C4-C8) et au moins un effluent oléfinique ayant un nombre d'atomes de carbones supérieur à 9 (C9+).
Dans ce cas, ledit effluent oléfinique C4-C8 est avantageusement envoyé dans la deuxième étape d'oligomérisation e) et ledit effluent oléfinique C9+ est avantageusement directement envoyé dans l'étape f) de fractionnement finale.
On définit ledit effluent oléfinique C4-C8 comme étant un effluent comprenant des composés oléfiniques repartis selon la distribution suivante : au moins 50% poids et de préférence au moins 70% poids de composés oléfiniques ayant un nombre d'atomes de carbone compris entre 4 et 8 et moins de 50% poids et de préférence moins de 30% poids d'autres composés oléfiniques, les pourcentages poids étant exprimés par rapport à la masse totale des oléfines présentes dans l'effluent oléfinique C4+.
De la même manière, on définit ledit effluent oléfinique C9+ comme étant un effluent comprenant des composés oléfiniques repartis selon la distribution suivante : au moins 50% poids et de préférence au moins 70% poids, de composés oléfiniques ayant un nombre d'atomes de carbone supérieur ou égal à 9 et comprenant également avantageusement moins de 50% poids et de préférence moins de 30% poids de composés ayant un nombre d'atomes de carbone inférieur à 9, les pourcentages poids étant exprimés par rapport à la masse totale des oléfines présentes dans l'effluent oléfinique C4+.
Dans ledit second mode de réalisation et dans le cas où l'étape de traitement du système catalytique homogène est mise en oeuvre selon la méthode 3 décrite ci-dessus, ladite séparation produit un premier effluent comprenant au moins une partie des composés oléfiniques C9+ et également le système catalytique homogène et en un deuxième effluent oléfinique exempt du système catalytique. Ladite séparation est ensuite suivie du traitement de l'effluent comprenant au moins une partie des composés C9+ et le système catalytique homogène par lavage acide et/ou basique ou par l'utilisation de masse de captation. Ledit effluent oléfinique comprenant au moins une partie des composés C9+, séparé du système catalytique homogène a l'issu du lavage acide et/ou basique est de préférence séché avant d'être envoyé dans étape f) de fractionnement finale.

Dans ce cas, ledit effluent oléfinique exempt du système catalytique, comprend de préférence au moins 80% poids et de préférence au moins 90% poids de composés oléfiniques C4+ et moins de 20% poids et de préférence moins de 10% poids d'éthylène (C2) n'ayant pas réagi lors de la première étape d'oligomérisation d), les pourcentages étant exprimés en pourcentages poids par rapport à la masse totale des oléfines contenues dans ledit effluent oléfinique exempt du système catalytique.
De préférence, lesdits composés oléfinique C4+ comprennent avantageusement au moins 80% poids, de préférence au moins 90% poids, de composés oléfiniques C4-C8 et moins de 20% poids et de préférence moins de 10% poids, de composés oléfiniques C9+, les pourcentages poids étant exprimés par rapport à la masse totale des oléfines présentes dans ledit effluent oléfinique C4+.
Selon une première variante, ledit effluent oléfinique exempt du système catalytique issu de l'étape de séparation est avantageusement directement envoyé dans la deuxième étape d'oligomérisation e), sans deuxième étape de séparation optionnelle intermédiaire.
Selon une deuxième variante, ledit effluent oléfinique exempt du système catalytique issu de l'étape de séparation subit ensuite avantageusement une deuxième étape de séparation optionnelle dans laquelle ledit effluent est avantageusement séparé en au moins un effluent C4+ et au moins un effluent léger oléfinique (C2-C4).
De préférence, la totalité dudit effluent léger oléfinique C2-C4 est recyclée dans la première étape d'oligomérisation d).

Ainsi, la charge envoyée en entrée de la deuxième étape d'oligomérisation e) comprend avantageusement, soit :
- la totalité de l'effluent issu de la première étape d'oligomérisation d) dans le cas où aucune étape de traitement du système catalytique homogène et/ou de séparation optionnelle n'est mise en oeuvre entre la première et la deuxième étape d'oligomérisation,
- soit au moins une partie de l'effluent issu de la première étape d'oligomérisation d), séparé de la totalité dudit effluent léger oléfinique (C2-C4) et/ou de la totalité dudit effluent oléfinique C9+, dans le cas où au moins une étape de traitement du système catalytique homogène et/ou de séparation optionnelles sont mises en oeuvre entre la première et la deuxième étape d'oligomérisation.
Au moins une partie de la coupe essence issus de l'étape de fractionnement finale f) peut avantageusement être également recyclée dans la deuxième étape d'oligomérisation e) du procédé selon l'invention et mélangé à la charge de l'étape e).

Conformément à l'étape e) du procédé selon l'invention, au moins une partie de l'effluent issu de la première étape d'oligomérisation d), éventuellement séparé de la totalité dudit effluent léger oléfinique (C2-C4) et/ou de la totalité de l'effluent oléfinique C9+, subit une deuxième étape e) d'oligomérisation en présence d'un catalyseur amorphe ou zéolithique, ledit catalyseur zéolithique ayant au moins des ouvertures de pores contenant 10 ou 12 atomes d'oxygènes (10MR ou 12MR), pour produire des bases hydrocarbonées distillats moyens.

Selon l'invention, la deuxième étape e) d'oligomérisation opère en présence d'un catalyseur amorphe ou comprenant au moins une zéolithe ayant au moins des ouvertures de pores contenant 10 ou 12 atomes d'oxygènes (10MR ou 12MR), et avantageusement choisie parmi les zéolithes de type aluminosilicate ayant un rapport global Si/Al supérieur à 10.

Selon un mode de réalisation préféré, le catalyseur utilisé dans la deuxième étape d'oligomérisation e) est un catalyseur amorphe comprenant et de préférence constitué d'un matériau minéral amorphe choisi parmi les silice-alumines et alumines silicées et de manière préférée les silices alumines.

Selon un autre mode de réalisation préféré, ledit catalyseur utilisé dans la deuxième étape d'oligomérisation e) comprend au moins une zéolithe choisie parmi les zéolithes de type aluminosilicate ayant un rapport global Si/Al supérieur à 10 et une structure de pores 10 ou 12MR, de préférence choisie parmi les zéolithes ZSM-5, ZSM-12, NU-86, Mordénite, ZSM-22, NU-10, ZBM-30, ZSM-48, ZSM-11, ZSM-57, IZM-2, ITQ-6 et IM-5, prises seules ou en mélange, de préférence parmi les zéolithes ZSM-5, NU-10 et ZBM-30, prises seules ou en mélange, de manière très préférée la zéolithe est la ZBM-30 et de manière encore plus préférée, la zéolithe est la ZBM-30 synthétisée en présence du structurant triéthylènetétramine.

La zéolithe mise en oeuvre dans le catalyseur utilisé dans l'étape e) du procédé selon l'invention peut avantageusement subir plusieurs post-traitements connus de l'homme de l'art tel que par exemple être modifiée par désalumination ou désilication selon toute méthode de désalumination, passivation de surface externe ou désilication connue de l'homme du métier, dans le but d'améliorer son activité et/ou sa stabilité.

Ledit catalyseur utilisé dans l'étape e) du procédé selon l'invention comprend également avantageusement au moins une matrice de type oxyde également appelé liant. On entend par matrice selon l'invention, une matrice amorphe ou mal cristallisée.
Ladite matrice est avantageusement choisie parmi les éléments du groupe formé par ies argiles (telles que par exemple parmi les argiles naturelles telles que le kaolin ou la bentonite), la magnésie, les alumines, les silices, les silice-alumines, les aluminates, l'oxyde de titane, l'oxyde de bore, la zircone, les phosphates d'aluminium, les phosphates de titane, les phosphates de zirconium, et le charbon. De préférence ladite matrice est choisie parmi les éléments du groupe formé par les alumines, les argiles et les silices, de manière plus préférée ladite matrice est choisie parmi les alumines, et de manière encore plus préférée ladite matrice est l'alumine gamma.

Les catalyseurs utilisés dans l'étape e) du procédé selon l'invention sont avantageusement mis en forme sous la forme de grains de différentes formes et dimensions. Ils sont avantageusement utilisés sous la forme d'extrudés cylindriques ou polylobés tels que bilobés, trilobés, polylobés de forme droite ou torsadée, mais peuvent éventuellement être fabriqués et employés sous la forme de poudre concassées, de tablettes, d'anneaux, de billes, de roues, de sphères. De préférence, lesdits catalyseurs sont sous forme d'extrudés de taille comprise entre 1 et 10 mm.

La deuxième étape e) d'oligomérisation est avantageusement mise en oeuvre dans au moins un réacteur en lit fixe.

La deuxième étape e) du procédé selon l'invention opère avantageusement à une température comprise entre 50 et 400°C, de préférence entre 100 et 350°C et de manière préférée entre 100 et 300°C, à une pression absolue comprise entre 2 et 15 MPa, de préférence entre 2 et 8 MPa et de manière préférée entre 3 et 8 MPa et à une vitesse pondérale horaire comprise entre 0,1 et 10 h⁻¹ et de préférence entre 0,4 et 5h⁻¹.

Conformément à l'invention, des bases hydrocarbonées distillats moyens (gazole et/ou kérosène) sont produites à l'issu de la deuxième étape e) d'oligomérisation.

L'effluent hydrocarboné oléfinique comprenant les bases hydrocarbonées distillats moyens produit lors de la deuxième étape d'oligomérisation e) est un effluent oléfinique comprenant au moins 80% poids et de préférence au moins 90% poids d'un effluent oléfinique C4+, et moins de 20%, de préférence moins de 10% d'éthylène (C2) n'ayant pas réagi, les pourcentages poids étant exprimés par rapport à la masse totale des oléfines contenues dans l'effluent produit.
Lesdits composés oléfiniques C4+ comprenent avantageusement moins de 50% poids et de préférence moins de 40% poids de composés oléfiniques C4-C8, et au moins 50% poids et de préférence au moins 60% poids de composés oléfiniques C9+, les pourcentages poids étant exprimés par rapport à la masse totale desdits composés oléfiniques C4+.

La deuxième étape d'oligomérisation e) permet donc la production d'un effluent enrichi en oléfines ayant un nombre d'atome de carbone supérieur ou égal à 9, par la conversion de des composés oléfiniques (C4-C8) entrant dans ladite étape en ces composés plus lourds.

Le procédé selon l'invention est un procédé flexible en ce sens que les conditions opératoires et le choix du catalyseur dans la deuxième étape e) d'oligomérisation permettent d'orienter la réaction vers l'un ou l'autre des produits cibles, à savoir dans un cas vers la production majoritaire d'une base hydrocarbonée de type gazole et dans l'autre d'une base hydrocarbonée de type kérosène.

Dans le cas où la production majoritaire de base hydrocarbonée de type gazole est plus particulièrement recherchée, la deuxième étape e) d'oligomérisation opère avantageusement en présence d'un catalyseur comprenant au moins une zéolithe choisie parmi les zéolithes de type aluminosilicate ayant un rapport global Si/Al supérieur à 10 et une structure de pores 10 ou 12MR et à une température comprise entre 200 et 300°C, à une pression comprise entre 3 et 7MPa et à une vitesse pondérale horaire comprise entre 0,1 et 5 h-1.

Dans le cas ou la production majoritaire de base hydrocarbonée de type kérosène est plus particulièrement recherchée, la deuxième étape e) d'oligomérisation opère avantageusement en présence d'un catalyseur amorphe, de préférence comprenant et de manière préférée constituée de silice alumine à une température comprise entre 100 et 300°C, à une pression comprise entre 2 et 6 MPa et à une vitesse pondérale horaire comprise entre 0,1 et 5 h⁻¹.

Conformément à l'invention, l'effluent issu de la deuxième étape d'oligomérisation e) comprenant les bases hydrocarbonées distillats moyens subit une étape de fractionnement finale f) dans au moins une colonne de distillation de manière à séparer lesdites bases en au moins deux coupes correspondants aux coupes essence et distillats moyens (gazole et/ou kérosène). Un effluent léger comprenant les composés C2 à C4 peut également être séparé pour être valorisé pur ou en mélange. Une fraction lourde ayant un point d'ébullition initial compris entre 350 et 370 °C peut également être avantageusement séparée. Ces produits cités ne sont nullement restrictifs.

Au moins une partie dudit effluent léger comprenant les composés C2 à C4 issue de l'étape de fractionnement f) peut avantageusement être recyclée dans la première étape d) d'oligomérisation du procédé selon l'invention.
Au moins une partie de la coupe essence issue de l'étape de fractionnement finale f) peut avantageusement être recyclées dans la deuxième étape e) d'oligomérisation du procédé selon l'invention.
Un des objectifs de la présente invention étant de maximiser le rendement en base distillats moyens et de préférence en base kérosène, ledit effluent léger et la coupe essence, non désirés, sont ainsi à nouveau oligomérisés respectivement dans l'étape d) et dans l'étape e) du procédé selon l'invention permettant l'augmentation de leur poids moléculaire et ainsi l'augmentation de leur point d'ébullition et leur compatibilité avec l'utilisation recherchée.

Au moins une partie et de préférence la totalité de la base distillats moyens (gazole et/ou kérosène) issu de l'étape de fractionnement finale f) subit avantageusement une étape d'hydrogénation des oléfines produites, pour les rendre incorporable au pool carburant.

De préférence, au moins une partie et de préférence la totalité de la base distillats moyens (gazole et/ou kérosène) issu de l'étape de fractionnement finale f) est mise en contact avec un gaz riche en hydrogène en présence d'un catalyseur comprenant au moins un métal du groupe VIII, avantageusement choisi parmi le palladium et le nickel pris seul ou en mélange, et un support avantageusement choisi parmi l'alumine, la silice ou la silice-alumine.
Le catalyseur mis en oeuvre dans l'étape optionnelle d'hydrogénation comprend une teneur en palladium avantageusement comprise entre 0,1 et 10 % poids et/ou une teneur en nickel, avantageusement comprise entre 1 et 60 % poids par rapport à la masse totale du catalyseur.
L'étape optionnelle d'hydrogénation opère avantageusement à une température comprise entre 100 et 250°C en entrée du réacteur, à une pression comprise entre 2 et 5 MPa et à une vitesse pondérale horaire comprise entre 0,05 et 8h-1.
On valide la performance de l'hydrogénation par une mesure du nombre de brome qui est avantageusement d'au plus 5 g Br/100g, dans le cas ou l'on souhaite saturer l'ensemble des composés insaturés présents dans la coupe à hydrogéner.
L'éffluent issu de l'étape optionnelle d'hydrogénation contient majoritairement des hydrocarbures valorisables et incorporables au pool kérosène et/ou gazole et de préférence kérosène.

Selon un mode de réalisation, au moins une partie dudit effluent issu de l'étape optionnelle d'hydrogénation peut avantageusement être recyclé soit dans la première étape d) d'oligomérisation de manière à constituer un diluant de la charge et à stabiliser ainsi le catalyseur et/ou peut avantageusement être introduit au niveau de l'étape optionnelle de séparation de façon à améliorer la séparation.

Une étape de séparation optionnelle suivant l'étape d'hydrogénation est avantageusement mise en oeuvre pour permettre le fractionnement en une coupe kérosène et/ou une coupe gazole et/ou une coupe ayant un point d'ébullition supérieur à 360°C.

### Description des figures

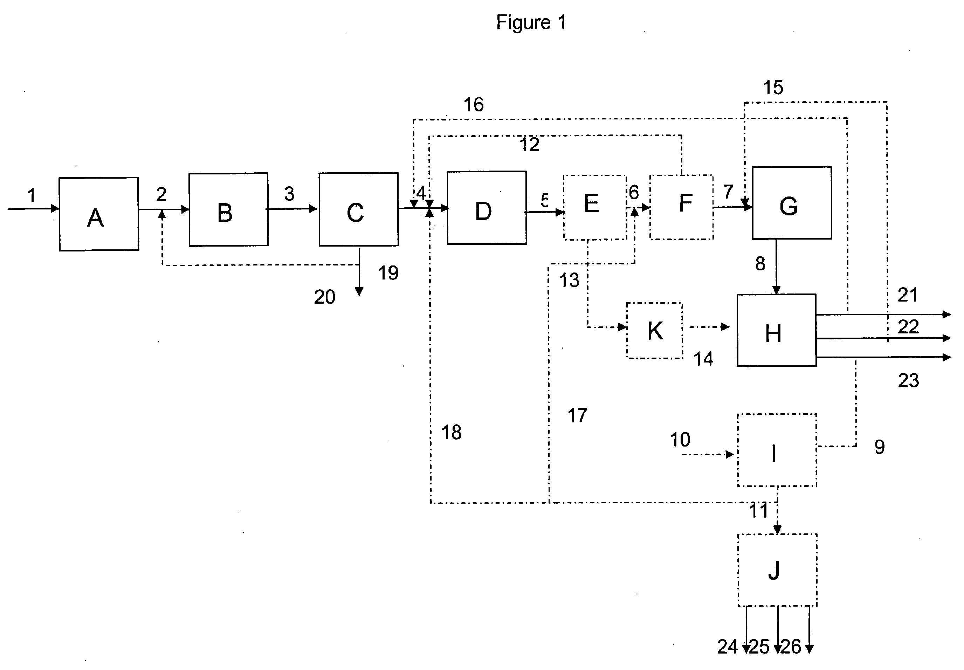

La figure 1 représente schématiquement le procédé de production de bases distillats moyens à partir de bioéthanol de la présente invention dans un deuxième mode de réalisation particulier incluant entre la première et la deuxième étape d'oligomérisation d) et e) une étape de traitement du système catalytique homogène selon la méthode 3 décrite ci dessus et d'une étape de séparation.

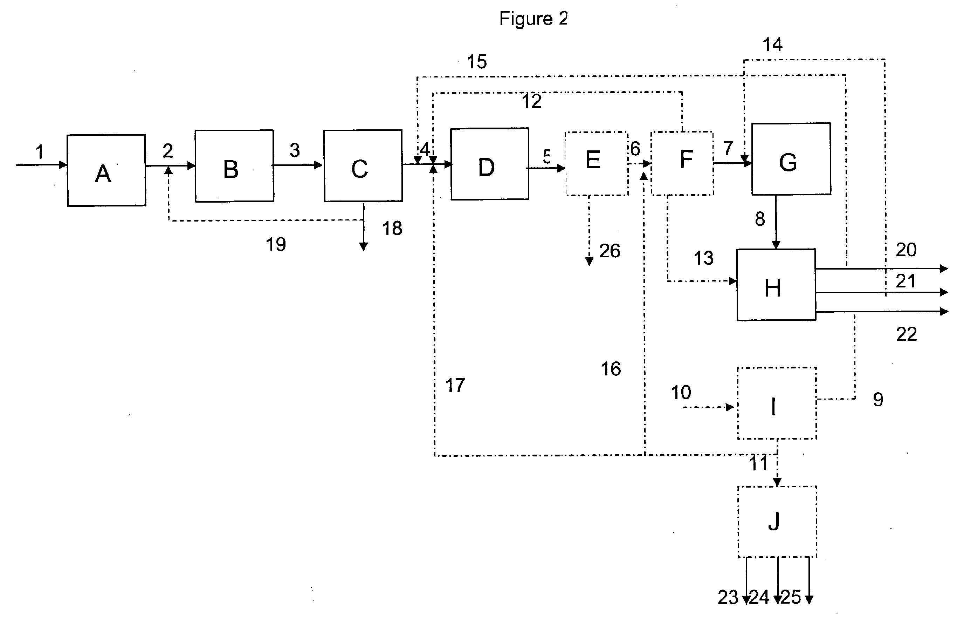

La figure 2 représente schématiquement le procédé de production de bases distillats moyens à partir de bioéthanol de la présente invention dans un mode de réalisation particulier incluant entre la première et la deuxième étape d'oligomérisation d) et e) une étape de traitement du système catalytique homogène par lavage acide et/ou basique, suivie d'une étape de séparation.

Sur la figure 1, la charge éthanol produite à partir de source renouvelable issue de la biomasse appelée bioéthanol est introduite dans une zone réactionnelle (A) via la conduite (1) dans laquelle ladite charge subit une étape de purification.
La charge ainsi purifiée (conduite 2) est ensuite envoyée dans une section réactionnelle (B) dans laquelle elle subit une étape b) de déshydratation pour produire un effluent majoritairement éthylènique et de l'eau. Ledit effluent majoritairement éthylènique est ensuite envoyé dans la section (C) via la conduite (3) dans laquelle il subit une étape c) de séparation de manière à séparer au moins une partie de l'eau formé lors de l'étape b) via la conduite (19) de l'effluent majoritairement éthylènique issu de l'étape b).
Selon une variante du procédé de l'invention représentée en pointillé, au moins une partie de l'effluent aqueux éliminé lors de l'étape c) de séparation est recyclée en amont de la section réactionnelle (B), servant de diluant de la charge bioéthanol purifiée, via la conduite (20). L'effluent majoritairement éthylènique issus de l'étape de séparation c) (conduite (4)) est envoyé dans une section réactionnelle (D) dans laquelle il subit une première étape d'oligomérisation pour produire au moins un effluent oléfinique léger comprenant au moins 80% poids d'oléfines ayant un nombre d'atomes de carbone supérieur ou égal à 4. Selon un mode de réalisation préféré représenté en pointillée, l'effluent issu de l'étape d) d'oligomérisation est ensuite envoyé (conduite (5)) dans une section de purification (E), dans laquelle il subit une séparation en un premier effluent comprenant au moins une partie des composés oléfiniques C9+ et également le système catalytique homogène (conduite 13) et en un deuxième effluent oléfinique exempt du système catalytique (conduite (6)), ladite séparation étant suivie du traitement de l'effluent comprenant au moins une partie des composés C9+ et le système catalytique homogène par lavage acide et/ou basique ou par l'utilisation de masse de captation dans la section K.
Ledit effluent oléfinique C9+ issu de l'étape de purification subit ensuite une étape de séchage non représenté sur la figure avant d'être envoyé dans la zone de fractionnement final (H) via la conduite (14). Dans le cas ou le traitement de l'effluent comprenant au moins une partie des composés C9+ et le système catalytique homogène est mis en oeuvre par lavage acide et/ou basique, l'effluent aqueux comprenant le système catalytique homogène est éliminé via la conduite (27).
Ledit effluent oléfinique exempt du système catalytique homogène et issu de la section de purification (E) via la conduite (6) subit ensuite une étape de séparation optionnelle dans laquelle ledit effluent est avantageusement séparé en au moins un effluent C4+ (conduite (7)) et au moins un effluent léger oléfinique (C2-C4) (conduite 12)) qui est recyclée dans la première étape d'oligomérisation d).
L'effluent issu de la zone de séparation optionnelle (F) est envoyé via la conduite (7) dans une zone réactionnelle (G) dans laquelle il subit une deuxième étape e) d'oligomérisation produisant des bases hydrocarbonées distillats moyens (conduite (8)).
L'effluent issu de l'étape e) d'oligomérisation est ensuite envoyé via la conduite (8) dans une zone de fractionnement (H) dans laquelle il est séparé en un effluent léger comprenant les composés C2-C4 via la conduite (21), en une coupe essence via la conduite (22), en une coupe distillats moyens (kérosène et/ou gazole via la conduite (23)).
Dans un mode de réalisation préféré représenté en pointillés, au moins une partie dudit effluent léger comprenant les composés C2 à C4 issus la zone de fractionnement (H) est recyclée dans la première étape d) d'oligomérisation via la conduite (16).
Dans un mode de réalisation préféré représenté en pointillés, au moins une partie de la coupe essence issue de la zone de fractionnement (H) est recyclée dans la deuxième étape e) d'oligomérisation via la conduite (15).
Dans un mode de réalisation préféré représenté en pointillés, au moins une partie des bases distillats moyens (conduite (9)) est envoyée dans une section (I) d'hydrogénation des oléfines produites, ladite section étant alimentée en hydrogène via la conduite (10).
L'effluent issu de l'étape optionnelle d'hydrogénation via la conduite (11) contient majoritairement des hydrocarbures valorisables et incorporable au pool kérosène et gazole. Selon un mode de réalisation représenté en pointillés, au moins une partie dudit effluent issu de l'étape optionnelle d'hydrogénation est envoyé via les conduites (17) et (18) respectivement dans les étapes d) d'oligomérisation de manière à de manière à constituer un diluant de la charge et à stabiliser ainsi le catalyseur et dans l'effluent issu de l'étape d) d'oligomérisation au niveau de l'étape optionnelle de séparation de façon à améliorer la séparation.
Au moins une partie dudit effluent issu de l'étape optionnelle d'hydrogénation est avantageusement envoyé via la conduite (11) dans une section (J) de séparation permettant le fractionnement en une coupe kérosène (conduite 24), en une coupe gazole (conduite 25) et en une coupe ayant un point d'ébullition supérieur à 360°C (conduite 26).

Sur la figure 2, la charge éthanol produite à partir de source renouvelable issue de la biomasse appelée bioéthanol est introduite dans une zone réactionnelle (A) via la conduite (1) dans laquelle ladite charge subit une étape de purification.
La charge ainsi purifiée (conduite 2) est ensuite envoyée dans une section réactionnelle (B) dans laquelle elle subit une étape b) de déshydratation en un effluent majoritairement éthylènique et en un effluent aqueux. Ledit effluent majoritairement éthylènique issu de l'étape b) est ensuite envoyé dans la section (C) via la conduite (3) dans laquelle il subit une étape c) de séparation de manière à séparer au moins une partie de l'effluent aqueux formé lors de l'étape b) via la conduite (18) de l'effluent majoritairement éthylènique issu de l'étape b).
Selon une variante du procédé de l'invention représentée en pointillé, au moins une partie de l'effluent aqueux éliminé lors de l'étape c) de séparation est recyclée en amont de la section réactionnelle (B), servant de diluant de la charge bioéthanol purifiée, via la conduite (19). L'effluent majoritairement éthylènique issus de l'étape de séparation c) (conduite (4)) est envoyé dans une section réactionnelle (D) dans laquelle il subit une première étape d'oligomérisation en au moins un effluent oléfinique léger comprenant au moins 80% poids d'oléfines ayant un nombre d'atomes de carbone supérieur ou égal à 4. Selon un mode de réalisation préféré représenté en pointillée, l'effluent issu de l'étape d) d'oligomérisation est ensuite envoyée (conduite (5)) dans une section de purification (E), dans laquelle il subit une étape de purification par traitement du catalyseur homogène par lavage acide ou basique de l'effluent oléfinique léger issu de la première étape d) d'oligomérisation, ledit catalyseur homogène étant séparé et éliminé dudit effluent oléfinique léger par séparation de la phase aqueuse dans laquelle il est solubilisé via la conduite (26).
Ledit effluent oléfinique sortant de l'étape de purification optionnelle via la conduite (6) subit ensuite une étape de séparation optionnelle dans la zone (F) dans laquelle ledit effluent est séparé en au moins un effluent C4+ (conduite (7) et au moins un effluent léger oléfinique (C2-C4) (conduite (12)) qui est recyclée dans la première étape d'oligomérisation d).
L'effluent oléfinique C4+ est séparé d'au moins une partie et de préférence la totalité de l'effluent oléfinique C9+ dans la zone (F) de séparation optionnelle via la conduite (13). Dans ce cas, ledit effluent oléfinique C4+, séparé d'au moins une partie et de préférence la totalité dudit effluent oléfinique C9+ est avantageusement envoyée via la conduite (7) dans la deuxième zone d'oligomérisation (G) et au moins une partie et de préférence la totalité dudit effluent oléfinique C9+ est avantageusement directement envoyé dans la zone (H) de fractionnement finale.
L'effluent issu de l'étape e) d'oligomérisation est ensuite envoyé via la conduite (8) dans une zone de fractionnement (H) dans laquelle il est séparé en un effluent léger comprenant les composés C2-C4 via la conduite (20), en une coupe essence via la conduite (21), en une coupe distillats moyens (kérosène et/ou gazole via la conduite (22)).
Dans un mode de réalisation préféré représenté en pointillés, au moins une partie dudit effluent léger comprenant les composés C2 à C4 issus de l'étape de fractionnement e) est recyclée dans la première zone (D) d'oligomérisation via la conduite (15).
Dans un mode de réalisation préféré représenté en pointillés, au moins une partie de la coupe essence issue de l'étape de fractionnement e) est recyclée dans la deuxième zone (G) d'oligomérisation via la conduite (14).

Dans un mode de réalisation préféré représenté en pointillés, au moins une partie des bases distillats moyens (conduite (9)) est envoyée dans une section (I) d'hydrogénation des oléfines produites, ladite section étant alimentée en hydrogène via la conduite (10). L'effluent issu de l'étape optionnelle d'hydrogénation via la conduite (11) contient majoritairement des hydrocarbures valorisables et incorporables au pool kérosène et gazole. Selon un mode de réalisation représenté en pointillés, au moins une partie dudit effluent issu de l'étape optionnelle d'hydrogénation est envoyé via les conduites (16) et (17) respectivement dans les étapes d) d'oligomérisation de manière à de manière à constituer un diluant de la charge et à stabiliser ainsi le catalyseur et dans l'effluent issu de l'étape d) d'oligomérisation au niveau de l'étape optionnelle de séparation de façon à améliorer la séparation.
Au moins une partie dudit effluent issu de l'étape optionnelle d'hydrogénation est avantageusement envoyé via la conduite (11) dans une section (J) de séparation permettant le fractionnement en une coupe kérosène (conduite 23), en une coupe gazole (conduite 24) et en une coupe ayant un point d'ébullition supérieur à 360°C (conduite 25).

L'exemple suivant illustre l'invention sans en limiter la portée.

### Exemple

### Exemple 1 selon l'invention :

### Description de la charge bioéthanol

La charge bioéthanol utilisée dans l'exemple est une charge de bioéthanol qui a été traitée par une succession d'étapes de distillation et de passages sur tamis moléculaires afin de répondre aux spécifications suivantes et dont la composition est donnée dans le tableau 1 :

**Tableau 1 : composition de la charge bioéthanol purifiée**

| Composition | Teneur en % poids |
|---|---|
| EtOH | 99% |
| Methanol | <0,05% |
| Butanol | <0,1% |
| Autres alcools | <0,05% |
| teneur totale en alcool autre que éthanol % poids | <0,2% |
| composés oxygénés autre que alcools | <0,1% |
| H2O | <1% |
| impuretés cationiques totale | <0,005% |
| impuretés anioniques totale | <0,005% |

### Étape b) : déshydratation de la charge purifiée

La charge purifiée subit ensuite une étape de déshydratation en éthylène et en eau en présence d'un catalyseur zéolithique C1.

### Préparation du catalyseur C1

Le catalyseur de déshydratation de l'étape b) est préparé comme décrit dans la demande de brevet US2009/088595.
Il s'agit d'un catalyseur zéolithique à base de ZSM-5.
On prépare une émulsion en introduisant dans un bécher d'un litre 244 g d'eau, 49 g de porogène constitué par de l'isane et 2,9 g de tensio-actif constitué par le galoryl. Le mélange est placé sous agitation à 500 tr/min pendant 15 min.
On prépare une suspension en introduisant dans un bécher de 4 litres 2198 g d'eau permutée et 69 g d'acide nitrique à 59,68 % poids, le mélange étant agité à 400 trs/min pendant 5 min. 450 g de PURAL SB3 (perte au feu = à 26,10 %) sont ensuite ajoutés et le mélange {eau permutée, acide nitrique et PURAL SB3} est agité à 1600 trs/min pendant 14 min. 332 g de zéolithe ZSM-5 sous forme H de rapport Si/Al égal à 140, commercialisée par la société Zeolyst, sont ensuite ajoutés au mélange {eau permutée, acide nitrique et PURAL SB3}, le mélange résultant est agité à 1600 trs/min pendant 3 min puis l'émulsion formée d'eau, d'isane et de galoryl est ajoutée audit mélange. L'ensemble est agité sous 1600 trs/min pendant 13 min puis la vitesse d'agitation est réduite à 625 trs/min pendant 70 min. La viscosité dudit mélange est ensuite mesurée au moyen d'un rhéomètre plan-plan pour un gradient de vitesse de cisaillement de 100 s⁻¹ et est égale 270 mPa.s.

Pour la mise en forme par coagulation en gouttes, on utilise une colonne en verre de 9,4 litres. On charge ladite colonne avec 7 litres d'une solution d'ammoniaque ayant une concentration égale à 28 g/l, 0,4 litre d'une solution d'ammonyl à 1 % massique et 0,7 litre d'isane. La colonne est surmontée d'un pot d'égouttage constitué de buses, chacune étant munie d'un orifice circulaire ayant un diamètre égal à 1 mm. On introduit la suspension dans ledit pot d'égouttage, le débit d'égouttage étant tel que 80 gouttelettes sont égouttées par minute et par buse. Les gouttelettes tombent ensuite dans la phase d'isane puis dans la phase d'ammoniaque à 28 g/l, l'interface phase d'isane - phase d'ammoniaque étant constituée d'ammonyl. Les billes ainsi obtenues sont placées dans un caisson ventilé à température ambiante pendant une nuit pour effectuer un premier séchage doux puis sont placées dans une étuve pendant une nuit à 100°C. Les billes séchées sont calcinées pendant 2 heures dans un four à moufle à 600°C. On obtient ainsi le catalyseur C1 dont les caractéristiques texturales et mécaniques sont données dans le tableau 2. Il présente une résistante mécanique telle que l'écrasement grain à grain (EGG) est égal à 26 N.

**Tableau 2 : caractéristiques texturales et mécaniques du catalyseur C1.**

| | C1 |
|---|---|
| surface BET (m²/g) | 321 |
| vol. poreux Hg (ml/g) | 0,41 |
| vol. macroporeux Hg (ml/g) | 0,12 |
| vol. mésoporeux Hg (ml/g) | 0,29 |
| taille billes sphériques (mm) | 1,8-2,2 |

L'étape b) du procédé selon l'invention est mise en oeuvre de manière à maximiser la production d'éthylène. L'étape b) déshydratation opère en présence du catalyseur zéolithique C1 décrit ci dessus et à une température de 400°C, à une pression de 0,1 MPa et à une vitesse pondérale horaire de 5 h-1.
L'étape b) déshydratation produit un effluent hydrocarboné majoritairement éthylènique comprenant de l'eau dont la répartition est donnée dans le tableau 3.

**Tableau 3 : répartition de l'effluent produit lors de l'étape b)**

| Répartition | % poids par rapport à la masse d'éthanol introduit dans l'étape b) |
|---|---|
| Eau | 39,5% pds |
| Effluent hydrocarboné | 60 % pds |
| Ethanol | 0.5% |

La composition de l'effluent majoritairement éthylènique produit lors de l'étape b) de déshydratation est mesurée par chromatographie en phase gazeuse (CPG), et donnée dans le tableau 4.

**Tableau 4 : Composition de l'effluent majoritairement éthylènique formé lors de la déshydratation de l'éthanol**

| | | % poids par rapport à la masse totale des composés hydrocarbonés et hydroxycarbonés formés |
|---|---|---|
| | Ethylène | 98,5% |
| | hydrocarbures ayant plus de 3 atomes de carbones | 1,5% |
| | Composés oxygénés | <0.1% |

La conversion de l'éthanol C2H5OH dans l'étape b) est de 99,5%. A partir d'1000 kg/h de bioéthanol purifié, 591 kg/h d'éthylène sont obtenus.

### Étape c) : séparation.

L'effluent majoritairement éthylènique issu de l'étape b) subit ensuite une série de séparation et de purification par passage dans une colonne de lavage à l'eau, puis par passage dans une colonne de lavage à la soude et dans des sécheurs de manière à séparer une partie de l'eau formée lors de l'étape b) de l'effluent éthylènique.

### Étape d) : oligomérisation.

L'effluent issu de l'étape c) de séparation est envoyé dans la première étape d'oligomérisation d) qui opère en présence d'un catalyseur homogène comportant un composé de nickel bivalent C2 décrit ci dessous et à une température de 45°C, à une pression de 3 MPa.

### Préparation du catalyseur C2 :

### Préparation de la solution de nickel :

Dans un ballon en verre de 2,5 litres muni d'un barreau magnétique pour l'agitation, on introduit 0,43 g d'éthyl-2 hexanoate de nickel à 13 % en poids de nickel, puis on purge soigneusement le ballon que l'on place sous atmosphère d'argon. On y introduit au moyen d'une aiguille de transfert 400 mL de pentane déoxygéné et séchée sur tamis moléculaire 3A, que l'on utilise dans la suite comme solvant. L'agitation permet de dissoudre le sel de nickel. On injecte ensuite une solution préparée à partir de 0,11 g d'acide trifluoroacétique complété à 100 g avec du pentane. L'ensemble est placé, toujours sous agitation, dans un bain thermostatique réglé à 30 °C. On dilue ensuite la solution obtenue avec du pentane de façon à ajuster la concentration en nickel à 0,1630 g Ni/Kg.

### Préparation de la solution de co-catalyseur EtAlCl₂ (EADC):

Le co-catalyseur est le dichloroéthylaluminium (EADC). Il est utilisé en solution dans le n-hexane. Une solution à 5 g EADC/Kg est utilisée.
La solution de nickel est injectée dans l'unité réactionnelle à l'aide d'une pompe (type LEWA). Le dichloroéthylaluminium est injecté séparément à l'aide d'une autre pompe.

### Réaction d'oligomérisation :

Le réacteur est un cylindre de 10 cm de diamètre et de 61 cm de hauteur. Le réacteur est muni d'une boucle de recirculation. La température dans le réacteur est maintenue constante et contrôlée par circulation d'eau dans une double enveloppe autour de la boucle de recirculation. La hauteur de liquide dans le réacteur est également contrôlée. L'entrée d'éthylène se fait en haut du réacteur et sous contrôle de débit. Les solutions de catalyseurs (Ni et EADC), conservées sous gaz inerte, sont injectées séparément dans la boucle de recirculation du réacteur sous contrôle de débit. Le rapport molaire de Al/Ni est fixé à 15. Le pourcentage d'EADC par rapport à la charge entrée est de 0,020% poids. La pression de marche dans le réacteur est de l'ordre de 3 MPa. La température de 45°C. L'éthylène entrée est pesé.
A la sortie du réacteur, l'analyse de l'effluent est réalisée sur la phase gazeuse par chromatographie en phase gazeuse et sur l'effluent liquide qui est pesé et analysé par chromatographie en phase gazeuse.

La composition de l'effluent oléfinique léger comprenant au moins 80% poids d'oléfines ayant un nombre d'atomes de carbone supérieur ou égal à 4 issu de l'étape b) est donnée dans le tableau 5.

**Tableau 5 : composition de l'effluent oléfinique léger issu de la première étape d'oligomérisation d)**

| | % poids par rapport à la masse totale des oléfines contenues dans l'effluent oléfinique produit à l'issu de l'étape d) |
|---|---|
| C2 | 4 |
| C4+ | 96 |
| | dont 96% de C4-C8 |
| | 4% de C9+ |

La coupe C4-C8 est composée de 54% poids d'oléfines en C4, de 36% poids d'oléfines en C6 et de 10% poids d'oléfines en C8.

### Traitement du système catalytique à l'issu de l'étape d) d'oligomérisation

L'effluent en sortie de d) est neutralisé par lavage avec une solution de soude à 20% poids dans un réacteur agité. La phase organique constituée par les produits liquides de la réaction est séparée par décantation et analysée. Le système catalytique homogène est séparé par élimination de la phase aqueuse.
Les impuretés de la charge comme l'eau et/ou les autres composés oxygénés sont majoritairement éliminés après passage sur tamis 3A et 13X commus de l'homme du métier.
A l'issue de l'étape de traitement du système catalytique, à partir d'1000 kg/h de bioéthanol purifié introduit sur l'étape b), 583 kg/h d'oléfines ayant 4 ou plus d'atomes de carbone sont obtenus.

L'effluent issu de l'étape de traitement du système catalytique est envoyé vers la deuxième étape d'oligomérisation e).

### Etape e) : deuxième étape d'oligomérisation.

L'étape e) d'oligomérisation opère en présence du catalyseur C3 décrit ci dessous et à une température de 150°C, à une pression absolue de 6,0 MPa et à une vitesse pondérale horaire de 0,7 h-1.

### Préparation du catalyseur C3 utilisé dans la deuxième étape d'oligomérisation e)

### Préparation du catalyseur C3

Une poudre d'hydroxyde d'aluminium est mélangée à un sol de silice préparé par échange sur résine décationisante, puis filtré sur résine de porosité 2. Les concentrations en sol de silice et en poudre d'hydroxyde d'aluminium sont ajustées de manière à obtenir une composition finale de 80% Al₂O₃ et 20% SiO₂. La mise en forme est réalisée en présence de 15% d'acide nitrique par rapport au produit anhydre. La pâte malaxée est alors extrudée au travers d'une filière de diamètre 1,4 mm. Les extrudés ainsi obtenus sont séchés à 120°C puis calcinés à 550°C.

La composition de l'effluent issu de la deuxième étape d'oligomérisation est décrite dans le tableau 6.

**Tableau 6: composition de l'effluent oléfinique issu de la deuxième étape d'oligomérisation e)**

| | % poids par rapport à la masse totale de l'effluent sortant |
|---|---|
| C2 | 4 |
| C4+ | 96 |
| | dont 32% de C4-C8 |
| | 68 % de C9+ |

L'effluent oléfinique C9+ présente une T95 inférieure à 360°C, la T95 étant la température à laquelle 95% des produits sont évaporés.

L'effluent issu de la seconde étape e) d'oligomérisation subit ensuite une étape f) de fractionnement de manière à le séparer en un effluent léger comprenant les composés C2-C4, une coupe essence et une coupe distillats moyens (gazole et kérosène).
Les rendements des différentes coupes sont indiqués dans le tableau 7.

**Tableau 7 : rendements**

| | % poids |
|---|---|
| C2-C4 | 12 |
| essence (50-150°C) | 23 |
| kérosène (150-280°C) | 51 |
| gazole (280-360°C) | 14 |

A l'issue de cette étape, à partir d'1000 kg/h de bioéthanol purifié introduit sur l'étape b), 309 kg/h de kérosène et 84,5 kg/h de diesel sont obtenus.

## Revendications

1. Procédé de production de bases hydrocarbonées distillats moyens et de préférence en base hydrocarbonée kérosène à partir d'une charge éthanol produite à partir de source renouvelable issue de la biomasse, ledit procédé comprenant au moins :
a) une étape de purification de ladite charge éthanol,
b) une étape de déshydratation de ladite charge éthanol purifiée issue de l'étape. a) en un effluent majoritairement éthylènique comprenant de l'eau, ladite étape opérant en présence d'un catalyseur acide amorphe ou d'un catalyseur acide zéolithique ,
c) au moins une étape de séparation de l'eau présente dans ledit effluent majoritairement éthylènique issu de l'étape b),
d) une première étape d'oligomérisation d'au moins une partie de l'effluent majoritairement éthylènique issu de l'étape c) en au moins un effluent oléfinique comprenant au moins 80% poids d'oléfines ayant un nombre d'atomes de carbone supérieur ou égal à 4, en présence d'un catalyseur homogène comprenant au moins un composé de nickel bivalent, les pourcentages poids étant exprimés en pourcentages poids par rapport à la masse totale des oléfines contenues dans ledit effluent oléfinique produit,
e) une deuxième étape d'oligomérisation d'au moins une partie de l'effluent issu de l'étape d) produisant des bases hydrocarbonées distillats moyens en présence d'un catalyseur amorphe ou zéolithique, ledit catalyseur zéolithique ayant au moins des ouvertures de pores contenant 10 ou 12 atomes d'oxygènes,
f) une étape de fractionnement de l'effluent issu de l'étape e) d'oligomérisation.

2. Procédé selon la revendication 1 dans lequel le catalyseur utilisé dans l'étape d) comprend au moins un composé de nickel bivalent, au moins un halogénure d'hydrocarbylaluminium et au moins un acide organique de Brönsted.

3. Procédé selon la revendication 2 dans lequel le composé de nickel bivalent est choisi parmi les sels de nickel bivalent du groupe octoate, éthyl-2-hexanoate, décanoate, stéarate, oléate, salicylate et hydroxydécanoate, pris seuls ou en mélange.

4. Procédé selon l'une des revendication 2 ou 3 dans lequel les halogénures d'hydrocarbylaluminium sont choisis parmi les dihalogénures d'hydrocarbyl-aluminium répondant à la formule AlRX2, dans laquelle R est un radical hydrocarbyle et X un halogène choisi parmi le fluor, le chlore, le brome et l'iode, pris seuls ou en mélange.

5. Procédé selon l'une des revendication 2 à 4 dans lequel les composés acides organiques de Brönsted sont choisis dans le groupe formé par les acides halogénocarboxyliques de formule RCOOH dans laquelle R est un radical alkyle halogéné renfermant au moins un atome d'halogène en alpha du groupe -COOH avec au total de 2 à 10 atomes de carbone.

6. Procédé selon l'une des revendications 1 à 5 dans lequel la totalité dudit effluent oléfinique produit lors de la première étape d) d'oligomérisation est directement envoyé dans la deuxième étape e) d'oligomérisation.

7. Procédé selon l'une des revendications 1 à 5 dans lequel, ledit effluent oléfinique produit lors de la première étape d) d'oligomérisation subit au moins une étape de traitement du système catalytique homogène et/ou au moins une étape de séparation avant d'être envoyé dans la deuxième étape e) d'oligomérisation.

8. Procédé selon la revendication 7 dans lequel ladite étape de traitement du système catalytique homogène est mise en oeuvre par l'utilisation de masse de captation ou par traitement de l'effluent oléfinique issu de la première étape d) d'oligomérisation, neutralisé ou non, et contenant le système catalyseur homogène par un acide et/ou une base.

9. Procédé selon la revendication 7 dans lequel ladite étape de traitement du système catalytique homogène est mise en oeuvre par la séparation dudit l'effluent oléfinique issu de la première étape d) d'oligomérisation, neutralisé ou non par une base, en un premier effluent comprenant au moins une partie des composés oléfiniques C9+ et également le système catalytique homogène et en un deuxième effluent oléfinique exempt du système catalytique, ladite séparation étant suivie du traitement de l'effluent comprenant au moins une partie des composés C9+ et le système catalytique homogène par lavage acide et/ou basique ou par l'utilisation de masse de captation.

10. Procédé selon l'une des revendications 7 à 9 dans lequel ladite étape de séparation est mise en oeuvre entre ladite étape de traitement du système catalytique homogène et la deuxième étape d'oligomérisation e), de manière à séparer ledit effluent oléfinique léger issu de la première étape d'oligomérisation d) en au moins un effluent oléfinique ayant un nombre d'atomes de carbone supérieur ou égal à 4 et en au moins un effluent léger oléfinique (C2-C4).

11. Procédé selon la revendication 10 dans lequel la totalité dudit effluent léger oléfinique C2-C4 est recyclée dans la première étape d'oligomérisation d).

12. Procédé selon l'une des revendications 1 à 10 dans lequel le catalyseur utilisé dans la deuxième étape d'oligomérisation e) comprend au moins une zéolithe choisie parmi les zéolithes ZSM-5, NU-10 et ZBM-30, prises seules ou en mélange.

13. Procédé selon l'une des revendications 1 à 11 dans lequel l'étape e) opère à une température comprise entre 50 et 400°C, à une pression absolue comprise entre 2 et 15MPa, et à une vitesse pondérale horaire comprise entre 0,1 et 50 h-1.

14. Procédé selon l'une des revendications 1 à 13 dans lequel au moins une partie de la base distillats moyens issu de l'étape de fractionnement f) subit une étape d'hydrogénation des oléfines produites, en présence d'un catalyseur comprenant au moins un métal du groupe VIII choisi parmi le palladium et le nickel pris seul ou en mélange, et un support choisi parmi l'alumine, la silice ou la silice-alumine.

15. Procédé selon la revendication 14 dans lequel une étape de séparation suivant l'étape d'hydrogénation est mise en oeuvre pour permettre le fractionnement en une coupe kérosène et/ou une coupe gazole et/ ou une coupe ayant un point d'ébullition supérieur à 360°C.
